# EUROPEAN PATENT SPECIFICATION

(11) **EP 1 800 681 B1**
(45) Date of publication and mention of the grant of the patent: **27.06.2012**
(21) Application number: 05799880.9
(22) Date of filing: 28.09.2005
(51) Int. Cl.: A61K 31/426

(54) **SOLID PHARMACEUTICAL COMPOSITION COMPRISING THE THIAZOLYL METHYL ESTER OF [5S-(5R*,8R*,10R*,11R*)]-10-HYDROXY-2-METHYL-5-(1-METHYLETHYL)-1-[2-1(1-METHYLETHYL)-4-THIAZOLYL]-3,6-DIOXO-8,11-BIS(PHENYLMETHYL)-2,4,7,12-TETRAAZATRIDECAN-13-OIC ACID AND PREPARATION METHOD THEREOF**
FESTE PHARMAZEUTISCHE ZUSAMMENSETZUNG MIT DEM THIAZOLYLMETHYL-ESTER VON [5S-(5R*,8R*,10R*,11R*)]-10-HYDROXY-2-METHYL-5-(1-METHYLETHYL)-1-[2-1(1-METHYLETHYL)-4-THIAZOLYL]-3,6-DIOXO-8,11-BIS(PHENYLMETHYL)-2,4,7,12-TETRAAZATRIDECAN-13-OONSÄURE UND HERSTELLUNGSVERFAHREN DAFÜR
COMPOSITION PHARMACEUTIQUE SOLIDE COMPRENANT DU THIAZOLYL METHYLESTER DE L'ACIDE [5S-(5R*,8R*,10R*,11R*)]-10 HYDROXY-2-METHYL-5-(1-METHYLETHYL)-1-[2-(1-METHYLETHYL)-4- THIAZOLYL]-3,6-DIOXO-8,11-BIS(PHENYLMETHYL)-2,4,7,12- TETRAAZATRIDECAN-13-OIQUE ET SON PROCEDE DE PREPARATION

(30) Priority: 28.09.2004 AR P040103504
(43) Date of publication of application: 27.06.2007
(73) Proprietor: Coll Farma S.L., 08960 San Just Desvern, Barcelona (ES)
(72) Inventor: ZINI, Elvira Beatriz, (C1062ABK) Ciudad de Buenos Aires (AR); RODRIGUEZ, Fernando, Javier, (B1602PI) Vicente Lopez, Provincia de Buenos Aires (AR); PAGANITTS, Cecilia, (CI408CZM) Ciudad de Buenos Aires (AR)
(74) Representative: Robba, Pierpaolo
(86) International application number: PCT/ES2005/000524
(87) International publication number: WO 2006/037827

(56) References cited:
- EP-A1- 0 608 850
- WO-A-95/09614
- WO-A1-00/25784
- WO-A1-2004/045586
- WO-A2-98/18477
- US-A1- 2004 024 031
- US-A1- 2005 143 404
- ENCYCLOPAEDIA BRITTANICA: 'alkaline-earth metal', [Online] Retrieved from the Internet: <URL:www.brittanica.com>
- ENCYCLOPAEDIA BRITTANICA: 'alkali metal', [Online] Retrieved from the Internet: <URL:www.brittanica.com>
- MACHINE TRANSLATION OF D6
- BAUER JOHN ET AL: "Ritonavir: An extraordinary example of conformational polymorphism", PHARMACEUTICAL RESEARCH (NEW YORK), vol. 18, no. 6, June 2001 (2001-06), pages 859-866, ISSN: 0724-8741
- ZELDIN ROBERT K ET AL: "Pharmacological and therapeutic properties of ritonavir-boosted protease inhibitor therapy in HIV-infected patients.", JOURNAL OF ANTIMICROBIAL CHEMOTHERAPY, vol. 53, no. 1, January 2004 (2004-01), pages 4-9, ISSN: 0305-7453
- Remington: "Farmacia 20 Ed.", 1 January 2000 (2000-01-01), Editorial medica Panamericana vol. tomo 1, page 425,
- 'EMEA Scientific Discussion on Norvir' 2001,
- "Handbook of Pharmaceutical Excipients - Magnesium Aluminium Silicate", 2006, Pharmaceutical Press * pages 418-421 *
- "Handbook of Pharmaceutical Excipients - Alcohol", 2006, Pharmaceutical Press * pages 18-20 *
- DEPARTMENT OF HEALTH & HUMAN SERVICES - FOOD AND DRUG ADMINISTRATION: 'Apticus prescribing information'
- DEPARTMENT OF HEALTH & HUMAN SERVICES - FOOD AND DRUG ADMINISTRATION: 'Lexiva prescribing information'

## Description

This invention refers to a solid pharmaceutical composition comprising the thiazolyl methyl ester of acid [5S-(5R*,8R*,10R*,11R*)]-10-hydroxi-2-methyl-5-(1-methylethyl)-1-[2-(1-methylethyl)-4-thiazo-lyl]-3,6-dioxo-8,11-bis(phenylmethyl)-2,4,7,12-tetraazatridecan-13-oic and a process to prepare same.

### BACKGROUND OF THE INVENTION

Active pharmaceutical ingredients may be administered in a variety of forms, for example in soft gel capsules, hard gel capsules, tablets, etc. In all these forms, the selection of the appropriate excipients is a key requirement to assure good bioavailability of the active pharmaceutical ingredient.

It has been determined that *in vitro* and *in vivo* HIV (human immunodeficiency virus) protease inhibiting compounds are useful to inhibit infections caused by this virus and are consequently useful for the treatment of AIDS. The compounds inhibiting the HIV protease are typically characterized by having low bioavailability and thus there is the concrete need of developing new and improved forms for their oral dosing.

In particular, compound [55-(5R*,8R*,10R*,11R*)]-10-hydroxi-2-methyl-5-(1-methyl-ethyl)-1-[2-(1-methylethyl)-4-thiazo-lyl]-3,6-dioxo-8, 11-bis(phenylmethyl)-2,4,7,12-tetra-azatridecan-13-oic, has proved to be especially effective as an HIV protease inhibitor. At present, it is most widely used as a pharmacologic booster. This is due to the fact that when administered jointly with other PI's (protease inhibitors) plasma levels are increased, causing, on some occasions, a reduction of the dose to be administered.

Ritonavir is the generic name given to compound 5*S*-(5*R**,8*R**,10R*, 11*R**)]-10-Hydroxy-2-methyl-5-(1-methylethyl)-1-[2-(1-methylethyl)-4-thiazolyl]-3,6-dioxo-8,11-bis(phenylmethyl)-2,4,7,12-tetraazatridecan-13-oic acid 5-thiazolylmethyl ester (synonyms: (2*S*,3*S*,5*S*)-5-[*N*-[*N*-[[N-methyl-*N*-[(2-isopropyl-4-thiazolyl)methyl]amino]carbonyl]valinyl] amino]-2-[*N*-[(5-thiazolyl)methoxycarbonyl]amino]-1,6-diphenyl-3-hydroxyhexane, A-84538 ; Abbott 84538; ABT-538, CAS Registry number: [155213-67-5]), which has the following structural formula:

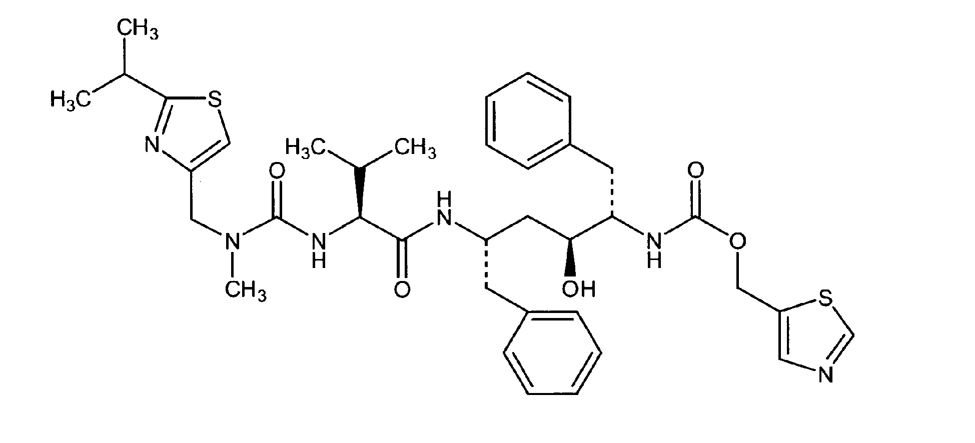

This compound was originally disclosed in the United States in the following patents:
US 5,541,206**:** "Retroviral protease inhibiting compounds" claiming as first priority document US1989000355945 of May 23, 1989. Patents US 5,635,523**:** "Retroviral protease inhibiting compounds", US 5,648,497**:** "Retroviral protease inhibiting compounds" and US 5,674,882**:** "Retroviral protease inhibiting compounds" are continuations of the above mentioned patent.
Patent US 5,846,987**:** "Retroviral protease inhibiting compounds" granted on December 08, 1998, claiming as first priority document US1992000998114 of December 29, 1992. Patent US 5,886,036**:** "Retroviral protease inhibiting compounds" continuation of the latter.

Furthermore, patent US 5,948,436**:** "Pharmaceutical composition" granted on September 07, 1999 claiming as first priority patent US19931993120886 of September 13, 1993, discloses a pharmaceutical composition which includes **RITONAVIR**.

Even when the usefulness of the compound mentioned above has been widely demonstrated, ritonavir has the inconvenience that it is not easily dissolved in aqueous media, which in the end may affect its bioavailability.
Generally, the literature has described that this compound has at least two polymorphic forms, named "polymorph 1" and "polymorph 2". Recently, patent application US 2004/0024031 A1 disclosed different crystalline forms of ritonavir (polymorphs 3, 4 and 5) and methods of treatment using them. Said document also discloses pharmaceutical compositions comprising the above mentioned crystals forms of ritonavir. Nevertheless, said patent application neither discloses how to obtain a solid pharmaceutical composition that solves the solubility inconveniences of ritonavir form 1, nor discloses the use of silicates of an alkaline metal in solid pharmaceutical compositions.

In order to try to overcome this inconvenience and achieve predictable and reproducible bioavailability of the active compound in the various batches of pharmaceutical products, the use of long chain fatty acids or the mixture of a long chain acid and an alcohol to which a surfactant may be optionally added has been suggested. For example, Argentine patent application P970105444 shows a pharmaceutical composition in a solution form which comprises: a) an HIV protease inhibitor or a combination of HIV inhibiting compounds, b) a pharmaceutically acceptable organic solvent which is made up of a pharmaceutically acceptable long chain fatty acid or a mixture of a pharmaceutically acceptable long chain fatty acid and a pharmaceutically acceptable alcohol, and optionally, c) a pharmaceutically acceptable surfactant.

According to what it is disclosed in said patent document, the composition (which is in solution form and could be utilized, for example in soft gel capsules) would have improved bioavailability when compared with the one which could be obtained with known solid pharmaceutical forms and standard suspensions.

Nevertheless, the formulation informed in the submission for Argentine patent P970105444 shows a serious inconvenient: it has undesirable secondary effects. This is due mainly to the fact that both long chain fatty acids and alcohols, which are essential to this formulation, show a significant hepatic intolerance. This inconvenience is further increased because patients to be treated with that formulation are those affected by the HIV virus.

On the other side, the preparation of soft gel capsules has, among others, the following disadvantage: it requires the use of more costly and complex technology, equipment and procedures. Additionally, the technology to prepare soft gel capsules needs lengthier and more complex procedures, which result in a higher manufacturing cost.

On the other hand, in order to obtain the approval by the regulatory authority to sell a pharmaceutical product, it is also necessary to demonstrate that the formulation of the active ingredient will yield a reproducible result in different patients. For example, in the case of solid formulations in tablet form it is necessary to demonstrate that they disintegrate and dissolve in a given period of time up to a given concentration.

Therefore, up to this date, ritonavir is sold only as a pharmaceutical product in soft gel capsules and in oral solution under the trade name *Norvir*®.
On the other hand, patent application WO 95/09614 discloses a composition which comprises silicon dioxide as an acceptable absorbent. Said document does not disclose the use of silicates of an alkaline metal in solid pharmaceutical compositions, in order to improve the solubility of ritonavir. At the same time, pharmaceutical compositions according to WO 95/09614 would comprise a high amount of added oils, which could cause gastric intolerance.
Moreover, patent application WO 00/25784 discloses the use of ritonavir to improve the pharmacokinetics of tripanavir. Said document does not disclose a pharmaceutical composition with and improved bioavailability of ritonavir that comprises a silicate of an alkaline metal, neither the use of said compound to enable ther penetration of ritonavir to the gastric fluids.
Besides, patent application JP 2006056781 discloses a process for preparing a pharmaceutical composition having improved bioavailability by mixing the drug in solution with a porous material, so that it can deposit on this material, solidify on it and take its form, thus giving more surface to improve its bioavailability. However JP 2006056781**,** though using ritonavir and calcium silicate in a preferred embodiment (see Example 11), explicitly refers to the use of fatty acids in order to make oral absorption possible. Moreover, JP 2006056781's process could be risky for ritonavir stability, because when it solidifies it could precipitate in a different polymorphic form, and the polymorphic form is critical for ritonavir bioavailability. In addition JP 2006056781 does not disclose a process where ritonavir is kept under solid state during the whole procedure.

Thus, there is the need to produce solid pharmaceutical compounds containing ritonavir, which are adequate for administration in human beings and animals, having chemical and physical properties in line with the requirements of national and international Drug Regulatory Authorities, and which do not have the disadvantages mentioned above.

Therefore, this invention provides a solid pharmaceutical form having adequate bioavailability, and adequate physical and chemical stability and which may be manufactured using a less costly and less complex technology than the one needed to manufacture soft gel capsules.

Surprisingly, we have discovered that now it is possible to obtain a solid pharmaceutical form containing ritonavir by means of a simple process that solves the problems of the previous art. This process involves the preparation of a dry or wet granulation which may be used to manufacture coated or uncoated tablets, hard gel capsules and extemporaneous suspension.

### BRIEF DESCRIPTION OF THE INVENTION

This invention is related with a solid pharmaceutical form of ritonavir and calcium silicate enabling the penetration of the gastrointestinal fluid in solid composition. In particular, this solid composition is free from both fatty acids and alcohol. The alcohol employed is evaporated during the process. The invention is defined as in the claims.

On the other hand, this invention is also related with a process to manufacture a solid pharmaceutical form of ritonavir which comprises a kneading step in which the speed and the time of addition of the solvent is thoroughly controlled. Especially, this process comprises a kneading step wherein the addition time of the granulation solvent is greater than 4 minutes, where the addition speed of the granulation solvent is within the range of about 300 mg/minute and about 500 mg/minute and where the relation between granulation solvent/dry component in the granulation is within the range of about 20 and about 60% w/w.

### DESCRIPTION OF THE INVENTION

This invention refers to a solid pharmaceutical form comprising particular excipients that improve wettability of ritonavir to help its dissolution. These excipients may be selected from among the group comprising silicates and silica oxide. Preferably, the ritonavir solid composition of the invention comprises ritonavir and calcium silicate, selected as the compound which enables the penetration of the gastrointestinal fluid in this composition. The selected calcium silicate has a density lower than 0.35 g/cm3. This density value is such that it allows obtaining a pharmaceutically acceptable formulation, since silicates or silica oxides having higher density values, with the addition of water, harden in a way that makes them impracticable to be used in oral compositions.

The simple fact that the composition contains neither fatty acids nor alcohol, allows for a better acceptance by the patient. This is due to the fact that, among others, the daily amount of fatty acids that a patient administered with Norvir® consumes causes the obvious undesirable effect of intestinal disorders, especially causing the frequent discharge of liquid feces (diarrhea).

Likewise, in addition to the important advantage mentioned above, the invention's solid composition containing ritonavir has the advantage of having improved stability when compared with liquid formulations of the prior art. This is due to the fact that, as known by those skilled in the art and as it is disclosed in the appropriate technical bibliography, there would be a greater possibility that undesirable reactions appear in the solution versus the solid state. In fact, the composition of the invention remains stable for at least 24 months kept in cold chain, that is, it remains stable for a longer time than the product sold with the *Norvir*® trade name. Furthermore, because of its greater stability, the invention composition remains stable at ambient temperature for at least 12 months. Finally, and no less importantly, is the fact that the composition is chemically and physically stable even when used as an active raw material in the so called ritonavir "polymorph 1".

Preferably, the solid composition of the invention is in the form of hard gel capsules, tablets or granulation for extemporaneous suspension. Notwithstanding, the coated tablet formulation is also preferable. According to the invention, the solid pharmaceutical composition may comprise ritonavir in combination with at least another therapeutically active substance different from the latter.

In particular, by means of the pharmaceutical composition of the invention, it is possible to obtain a solid composition of ritonavir with improved bioavailability, which may be bioequivalent to, or have better bioavailability than the solution compositions containing fatty acids of the prior art, an at the same time having a smaller final size, which would also enable a better acceptance by the patients.

Especially, this invention provides solid formulations of ritonavir with at least 75% dissolution after 15 minutes (dissolution media used: 500 ml of hydrochloric acid 0.1 N at 100 rpm in number 1 dissolution equipment (USP)).

The solid pharmaceutical composition of the invention may be obtained by means of a safe procedure in which ritonavir is wet and dry granulated with the adequate excipients which facilitate moisturing.

Therefore this is another object of the invention: a process to prepare a solid pharmaceutical form of ritonavir comprising the steps of granulating and mixing and, optionally an intermediate mixing step. Depending on the final pharmaceutical form which is intended to prepare, the process may comprise compression, coating, capsule filling and/or dosing.

Particularly, another object of the invention is to provide a process to prepare a solid pharmaceutical form of ritonavir comprising a kneading step in which the solvent addition speed and time are monitored thoroughly. Preferably, the invention procedure comprises a kneading step wherein the addition time of the granulation solvent is greater than 4 minutes, wherein the addition speed of the granulation solvent is within the range of about 300 mg/minute and about 500 mg/minute and where the granulation solvent/dry component ratio in the granulation is within the range of about 20 and about 60 % w/w. It would be even more preferable that in the kneading step the granulation solvent/dry component ratio in the granulation were within the range of about 35 and about 40% p/p.

Preferably, for better results, the kneading step should be made during a period of time of about 5 and about 7 minutes. The solvent to be used should be a mixture of ethanol water 70-30 p/p, in which the agglutinant was previously dissolved.

While a certain number of presentation forms of this invention are described herein, it is evident that the examples cited may be modified in order to yield other solid compositions using the formulations and procedures of this invention. Therefore it becomes evident that the scope of application of this invention includes all alternative solid forms and variations defined in the description stated above and in the examples and the claims included below. Thus the invention shall not be considered as limited to specific formulations which are included here as examples.

### EXAMPLES OF PRODUCTION PROCESSES ENTAILED IN THE FOLLOWING INVENTION:

### Example 1:

### Solid pharmaceutical composition of ritonavir:

| ***Component*** | ***Unit Formula*** |
|---|---|
| Ritonavir | 100,0 mg |
| Coloidal silica dioxide | 1,2 mg |
| Kollidon CL | 20,0 mg |
| Sodium Lauril Sulfate | 10,0 mg |
| Tablettose 80 | 244,8 mg |
| Starch 1500 | 20,0 mg |
| Magnesium Stearate | 4,0 mg |
| Granulation Solvent | Not applicable (direct compression) |
| *Core Total* | *400*,*0 mg* |

### Example 2:

### Solid pharmaceutical composition containing 100 mg of ritonavir:

| ***Component*** | ***Unit Formula*** |
|---|---|
| Ritonavir | 100,0 mg |
| Calcium Silicate | 46,5 mg |
| Kollidon CL | 46,5 mg |
| Kollidon VA 64 | 4,5 mg |
| Magnesium Stearate | 2,5 mg |
| Granulation Solvent | Ethanol 96°: Water (70:30) |
| *Core Total* | *200,0 mg* |

### Example 3:

| ***Component*** | ***Unit Formula*** |
|---|---|
| Ritonavir | 100,0 mg |
| Calcium Silicate | 46,5 mg |
| Kollidon CL | 46,5 mg |
| Kollidon VA 64 | 4,5 mg |
| Magnesium Stearate | 2,5 mg |
| Granulation Solvent | Ethanol 96°: Water (70:30) |
| *Core Total* | *200,0 mg* |

### Example 4:

| ***Component*** | ***Unit Formula*** |
|---|---|
| Ritonavir | 100,0 mg |
| Calcium Silicate | 186,5 mg |
| Kollidon CL | 186,5 mg |
| Kollidon VA 64 | 20,0 mg |
| Corn Starch | 492,0 mg |
| Magnesium Stearate | 15,0 mg |
| Granulation Solvent | Ethanol 96°: Water (70:30) |
| *Core Total* | *1000,0 mg* |

### Example 5:

| ***Component*** | ***Unit formula*** |
|---|---|
| Ritonavir | 100,0 mg |
| Calcium Silicate | 186,5 mg |
| Kollidon CL | 186,5 mg |
| Lactose Monohydrate | 492,0 mg |
| PVP K30 | 20,0 mg |
| Magnesium Stearate | 15,0 mg |
| Granulation Solvent | Ethanol 96°: Water (70:30) |
| *Core Total* | *1000,0 mg* |

### Example 6:

| ***Component*** | ***Unit Formula*** |
|---|---|
| Ritonavir | 100,0 mg |
| Calcium Silicate | 186,5 mg |
| Kollidon CL | 186,5 mg |
| Lactose Monohydrate | 492,0 mg |
| Kollidon VA 64 | 20,0 mg |
| Corn Starch | 246,0 mg |
| Magnesium Stearate | 15,0 mg |
| Granulation Solvent | Ethanol 96°: Water (70:30) |
| *Core Total* | *1000,0 mg* |

### Example 7:

| ***Component*** | ***Unit Formula*** |
|---|---|
| Ritonavir | 100,0 mg |
| Calcium Silicate | 186,5 mg |
| Kollidon CL | 186,5 mg |
| Lactose Monohydrate | 123,0 mg |
| Kollidon VA 64 | 20,0 mg |
| Corn Starch | 369,0 mg |
| Magnesium Stearate | 15,0 mg |
| Granulation Solvent | Ethanol 96°: Water (70:30) |
| *Core Total* | *1000,0 mg* |

### Example 8

| ***Component*** | ***Unit Formula*** |
|---|---|
| Ritonavir | 100,0 mg |
| Calcium Silicate | 186,5 mg |
| Kollidon CL | 186,5 mg |
| Kollidon VA 64 | 20,0 mg |
| Corn Starch | 492,0 mg |
| Magnesium Stearate | 15,0 mg |
| Granulation Solvent | Ethanol 96°: Water (70:30) |
| *Core Total* | *1000,0 mg* |

### Example 9 Comparison Abbott product

| ***Component*** | ***Unit Formula*** |
|---|---|
| Ritonavir | 100,0 mg |
| Etanol | 136,75 mg |
| Polyoxyl Castor Oil 35 | 120,0 mg |
| Propylene Glycol | 50,0 mg |
| Medium-Sized Chain | 36,0 triglyceride mg |
| Polysorbate 80 and 30 | 36,0 mg |
| Citric Acid | 30,75 mg |
| Saturated poly glycolized | 260,0 mg |
| Glycerides | |
| *Capsule Total* | *769, 5 mg* |

### Example 10: Dissolution Study

Comparative study of pharmaceutical compositions of ritonavir according to the invention and the commercial product Norvir®

| | |
|---|---|
| 1) Condition "1": | Media: Water |
| | Apparatus: Basket |
| | Speed: 100 rpm |
| 2) Condition "2": | Media: HCl 0,1N |
| | Apparatus: Paddles |
| | Speed: 50 rpm |

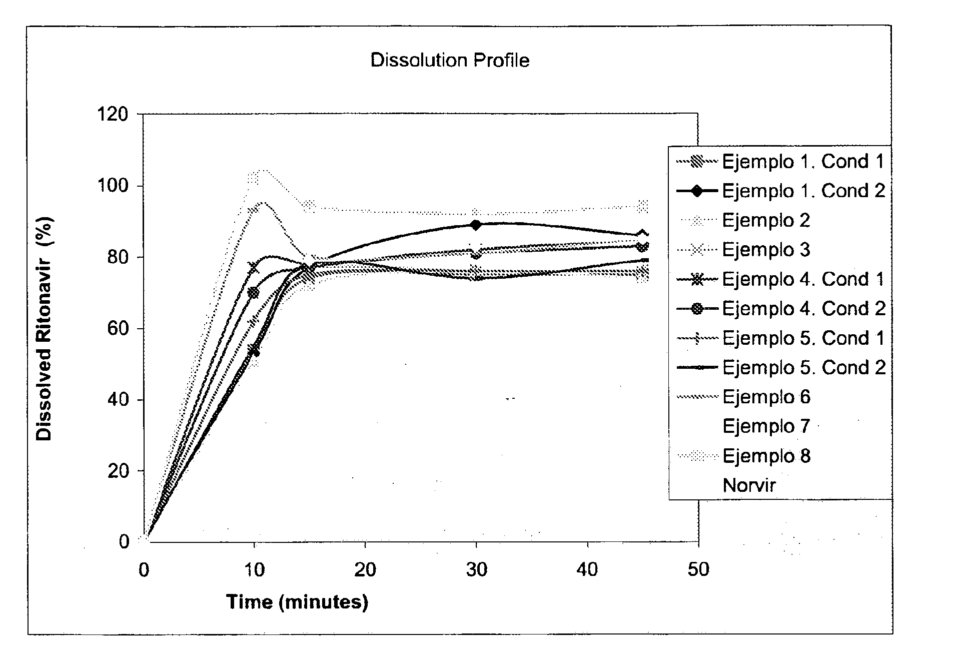

### Example 11:

Process to prepare ritonavir granulate according to the invention.

Ritonavir is sifted to homogeneize the particle size to a size of between 100 µm and 1000 µm. A quantity of calcium silicate, polyvinyl pyrrolidone and lactose monohydrate is sifted using the same mesh. The elements are mixed in an adequate powder mixer for the time and with the speed sufficient to ensure homogeneity and prevent the formation of very fine powders.

The mixture is granulated using an adequate piece of equipment, using crospovidone dissolved in a mixture of ethyl alcohol-water. This proportion may vary depending on the granulator used. The granulation variables are adjusted to obtain granulation whose specific surface guarantees good wettability.

The granulate is dried using either a fluid bed or a static dryer, depending on the granulation technology of choice.

The resulting granulate is mixed with polyvinyl pyrrolidone and magnesium stearate. If appropriate, a preservative agent, a sweetener, a flavoring agent and a coloring agent may be added.

This is later sifted using an adequate mesh, which will be selected depending on the pharmaceutical form in which this granulate will be used.

### Example 12:

Process to prepare ritonavir granulate according the invention.

Ritonavir is sifted to homogeneize the particle size to a size of between approximately 100 µm and approximately 1000 µm. A quantity of calcium silicate, polyvinyl pyrrolidone and corn starch is sifted using the same mesh. The elements are mixed in an adequate powder mixer for the time and with the speed sufficient to ensure homogeneity and prevent the formation of very fine powders

The mixture is granulated using an adequate piece of equipment, using crospovidone dissolved in a mixture of ethyl alcohol-water. This proportion may vary depending on the granulator used. The granulation variables are adjusted to obtain a granulation whose specific surface guarantees good wettability.

The granulate is dried using either a fluid bed or a static dryer, depending on the granulation technology of choice.

The resulting granulate is mixed with polyvinyl pyrrolidone and magnesium stearate. If appropriate, a preservative agent, a sweetener, a flavoring agent and a coloring agent may be added.

This is later sifted using an adequate mesh, which will be selected depending on the pharmaceutical form in which this granulate will be used.

### Example 13: Stability Study

A physical stability study was performed in which the pharmaceutical compositions were maintained in keeping with examples 1 through 9 for six months under stress conditions at a temperature of 40°C and 75% relative humidity. No significant changes were observed in the chemical composition, physical appearance or dissolution of the compositions.

### Example 14: Ritonavir 50 mg + Lopinavir 200 mg

| DESCRIPTION | UNIT | QUANTITY |
|---|---|---|
| Ritonavir | mg | 50,0 |
| Lopinavir | mg | 200,0 |
| Calcium Silicate | mg | 60,0 |
| Kollidon CL | mg | 60,0 |
| Corn Starch | mg | 140,0 |
| Kollidon VA 64 | mg | 10,0 |
| Microcrystalline Cellulose PH 200 | mg | 75,0 |
| Magnesium Stearate | mg | 5,0 |
| Core Weight | mg | 600,0 |

### Example 15: Ritonavir 100 mg + Lopinavir 400 mg

| DESCRIPTION | UNIT | QUANTITY |
|---|---|---|
| Ritonavir | mg | 100,0 |
| Lopinavir | mg | 400,0 |
| Calcium Silicate | mg | 120,0 |
| Kollidon CL | mg | 120,0 |
| Corn Starch | mg | 280,0 |
| Kollidon VA 64 | mg | 20,0 |
| Microcrystalline Cellulose PH 200 | mg | 150,0 |
| Magnesium Stearate | mg | 10,0 |
| Core Weight | mg | 1200,0 |

### Example 16: Ritonavir 50 mg + Saquinavir 500 mg

| DESCRIPTION | UNIT | QUANTITY |
|---|---|---|
| Ritonavir | mg | 50,0 |
| Saquinavir | mg | 500,0 |
| Calcium Silicate | mg | 120,0 |
| Kollidon CL | mg | 120,0 |
| Corn Starch | mg | 260,0 |
| Kollidon VA 64 | mg | 20,0 |
| Microcrystalline Cellulose PH 200 | mg | 120,0 |
| Magnesium Stearate | mg | 10,0 |
| Core Weight | mg | 1200,0 |

### Example 17: Ritonavir 50 mg + Indinavir 400 mg

| DESCRIPTION | UNIT | QUANTITY |
|---|---|---|
| Ritonavir | mg | 50,0 |
| Indinavir | mg | 400,0 |
| Calcium Silicate | mg | 130,0 |
| Kollidon CL | mg | 130,0 |
| Corn Starch | mg | 300,0 |
| Kollidon VA 64 | mg | 20,0 |
| Microcrystalline Cellulose PH 200 | mg | 160,0 |
| Magnesium Stearate | mg | 10,0 |
| Core Weight | mg | 1200,0 |

### Example 18: Ritonavir 100 mg + Tenofovir 300 mg

| DESCRIPTION | UNIT | QUANTITY |
|---|---|---|
| Ritonavir | mg | 100,0 |
| Tenofovir | mg | 300,0 |
| Calcium Silicate | mg | 130,0 |
| Kollidon CL | mg | 130,0 |
| Corn Starch | mg | 310,0 |
| Kollidon VA 64 | mg | 20,0 |
| Microcrystalline Cellulose PH 200 | mg | 200,0 |
| Magnesium Stearate | mg | 10,0 |
| Core Weight | mg | 1200,0 |

### Example 19: Ritonavir 100 mg + Amprenavir 600 mg

| DESCRIPTION | UNIT | QUANTITY |
|---|---|---|
| Ritonavir | mg | 100,0 |
| Amprenavir | mg | 600,0 |
| Calcium Silicate | mg | 120,0 |
| Kollidon CL | mg | 120,0 |
| Corn Starch | mg | 200,0 |
| Kollidon VA 64 | mg | 20,0 |
| Microcrystalline Cellulose PH 200 | mg | 130,0 |
| Magnesium Stearate | mg | 10,0 |
| Core Weight | mg | 1300,0 |

### Example 20: Ritonavir 100 mg + Fosamprenavir 700 mg

| DESCRIPTION | UNIT | QUANTITY |
|---|---|---|
| Ritonavir | mg | 100,0 |
| Fosamprenavir | mg | 700,0 |
| Calcium Silicate | mg | 120,0 |
| Kollidon CL | mg | 120,0 |
| Corn Starch | mg | 100,0 |
| Kollidon VA 64 | mg | 20,0 |
| Microcrystalline Cellulose PH 200 | mg | 130,0 |
| Magnesium Stearate | mg | 10,0 |
| Core Weight | mg | 1300,0 |

### Example 21: Ritonavir 100 mg + Atazanavir 400 mg

| DESCRIPTION | UNIT | QUANTITY |
|---|---|---|
| Ritonavir | mg | 100,0 |
| Atazanavir | mg | 400,0 |
| Calcium Silicate | mg | 120,0 |
| Kollidon CL | mg | 120,0 |
| Corn Starch | mg | 280,0 |
| Kollidon VA 64 | mg | 20,0 |
| Microcrystalline Cellulose PH 200 | mg | 150,0 |
| Magnesium Stearate | mg | 10,0 |
| Core Weight | mg | 1200,0 |

### Example 22: Ritonavir 200 mg + Tripanavir 500 mg

| DESCRIPTION | UNIT | QUANTITY |
|---|---|---|
| Ritonavir | mg | 200,0 |
| Tripanavir | mg | 500,0 |
| Calcium Silicate | mg | 120,0 |
| Kollidon CL | mg | 120,0 |
| Corn Starch | mg | 200,0 |
| Kollidon VA 64 | mg | 20,0 |
| Microcrystalline Cellulose PH 200 | mg | 130,0 |
| Magnesium Stearate | mg | 10,0 |
| Core Weight | mg | 1300,0 |

## Claims

1. A solid pharmaceutical composition comprising a solid pharmaceutical form of ritonavir and calcium silicate enabling the penetration of the gastrointestinal fluid in said composition, said composition being free from both fatty acids and alcohol **characterized in that** ritonavir compound is in its polymorph 1 form.

2. A solid pharmaceutical composition according to claim 1, **characterized in that** it is a coated or uncoated tablet, a hard gel capsule or an extemporaneous suspension.

3. A solid pharmaceutical composition according to any one of the previous claims **characterized in that** it further comprises one or more excipients selected from among Kollidon CL, Kollidon VA 64, corn starch, sodium lauril sulfate, Tablettose 80, starch 1500, magnesium stearate, lactose monohydrate and PVP K30.

4. A solid pharmaceutical composition according to any one of the previous claims **characterized in that** it further comprises one or more excipients selected from among preservatives, sweeteners, flavoring agents and coloring agents.

5. A process for the manufacture of a solid pharmaceutical composition according to claim 1 **characterized by** comprising a kneading step wherein the addition time of the granulation solvent is greater than 4 minutes, wherein the speed of addition of the granulation solvent is within the range of about 300 mg/minute and about 500 mg/minute and wherein the ratio granulation solvent/dry component in the granulate is within the range of about 20 and about 60% p/p.

6. A process for the manufacture of a solid pharmaceutical composition according to claim 5, **characterized in that** in the kneading step, the relation granulation solvent / dry component in the granulate is within the range of about 35 and about 40 % p/p.

7. A process for the manufacture of a solid pharmaceutical composition according to claim 5, **characterized in that** the kneading step is performed during a period of about 5 and about 7 minutes.

8. A process for the manufacture of a solid pharmaceutical composition according to claim 5, **characterized in that** a mixture of ethanol/water 70-30 w/w, in which the agglutinant was previously dissolved, is used as granulation solvent.

9. A process for the manufacture of a solid pharmaceutical composition according to claim 8, **characterized in that** a mixture of ethanol/water 70/30 p/p, in which kollidon 64 was previously dissolved, is used as granulation solvent.

10. A solid pharmaceutical composition according to any of claims 1 through 4, **characterized in that** it comprises ritonavir in a combination with at least one therapeutically active substance which is different from ritonavir.

11. A solid pharmaceutical composition according to claim 10, **characterized in that** said therapeutically active substance different from ritonavir is selected from among lopinavir, saquinavir, indinavir, tenofovir, amprenavir, fosamprenavir, atazanavir and tripanavir.

## Patentansprüche

1. Eine feste pharmazeutische Zusammensetzung, welche aus einer festen Darreichungsform von Ritonavir und Kalzium-Silikat besteht, die das Eindringen der gastrointestinalen Flüssigkeit in der besagten Zusammensetzung ermöglicht, wobei die besagte Zusammensetzung frei von Fettsäuren und Alkohol ist, **dadurch gekennzeichnet, dass** die Ritonavir Verbindung in ihrer polymorphen Form 1 ist.

2. Eine feste pharmazeutische Zusammensetzung gemäß Anspruch 1, **dadurch gekennzeichnet, dass** sie eine beschichtete oder unbeschichtete Tablette, eine Gelkapsel oder eine unvorbereitete Suspension ist.

3. Eine feste pharmazeutische Zusammensetzung gemäß irgendeinem der vorangehenden Ansprüche, **dadurch gekennzeichnet, dass** sie auch einen oder mehrere von den folgenden Hilfsstoffen enthält: Kollidon CL, Kollidon VA 64, Maisstärke, Natriumlaurylsulfat, Tablettose 80, Stärke 1500, Magnesiumstearat, Laktose-Monohydrat und PVP K30.

4. Eine feste pharmazeutische Zusammensetzung gemäß irgendeinem der vorangehenden Ansprüche, **dadurch gekennzeichnet, dass** sie auch einen oder mehrere von den folgenden Hilfsstoffen enthält: Konservierungs-, Süß-, Aroma- und Farbstoffe.

5. Ein Verfahren für die Herstellung einer festen pharmazeutischen Zusammensetzung gemäß Anspruch 1, **dadurch gekennzeichnet, dass** es eine Knete-Phase umfasst, wobei die Hinzufügungszeit des Granulationslösungsmittels über 4 Minuten beträgt, wobei die Hinzufügungsgeschvvindigkeit des Granulationslösungsmittels sich im Bereich von zwischen etwa 300 mg/Minute und etwa 500 mg/Minute befindet und wobei das Verhältnis Granulationslösungsmittel/trockene Komponente des Granulats sich im Bereich von zwischen etwa 20 und etwa 60 % Gew./Gew. befindet.

6. Ein Verfahren für die Herstellung einer festen pharmazeutischen Zusammensetzung gemäß Anspruch 5, **dadurch gekennzeichnet, dass** in der Knete-Phase das Verhältnis Granulationslösungsmittel/trockene Komponente des Granulats sich im Bereich von zwischen etwa 35 und etwa 40 % Gew./Gew. befindet.

7. Ein Verfahren für die Herstellung einer festen pharmazeutischen Zusammensetzung gemäß Anspruch 5, **dadurch gekennzeichnet, dass** die Knete-Phase in einer Zeitspanne von zwischen etwa 5 und etwa 7 Minuten durchgeführt wird.

8. Ein Verfahren für die Herstellung einer festen pharmazeutischen Zusammensetzung gemäß Anspruch 5, **dadurch gekennzeichnet, dass** eine Mischung aus Ethanol/Wasser 70/30 Gew./Gew., in welcher zuvor das Bindemittel aufgelöst wurde, als Granulationslösungsmittel benutzt wird.

9. Ein Verfahren für die Herstellung einer festen pharmazeutischen Zusammensetzung gemäß Anspruch 8, **dadurch gekennzeichnet, dass** eine Mischung aus Ethanol/Wasser 70/30 Gew./Gew., in welcher zuvor Kollidon 64 aufgelöst wurde, als Granulationslösungsmittel benutzt wird.

10. Eine feste pharmazeutische Zusammensetzung gemäß irgendeinem der Ansprüche 1 bis 4, **dadurch gekennzeichnet, dass** sie Ritonavir in Verbindung mit vvenigstens einer therapeutisch aktiven Substanz enthält, die sich vom Ritonavir unterscheidet.

11. Eine feste pharmazeutische Zusammensetzung gemäß Anspruch 10, **dadurch gekennzeichnet, dass** die besagte therapeutisch aktive Substanz, die sich vom Ritonavir unterscheidet, unter den folgenden ausgewählt wird: Lopinavir, Saquinavir, Indinavir, Tenofovir, Aprenavir, Fosamprenavir, Atazanavir und Tripanavir.

## Revendications

1. Une composition pharmaceutique solide comprenant une forme pharmaceutique solide du ritonavir et du silicate de calcium, qui permet la pénétration du fluide gastro-intestinal dans ladite composition, ladite composition étant exempte tant d'acides gras que d'alcool, **caractérisée en ce que** le composé ritonavir est dans sa forme polymorphe 1.

2. Une composition pharmaceutique solide selon la revendication 1, **caractérisée en ce qu'**elle est un comprimé pelliculé ou non pelliculé, une gélule dure ou de la poudre pour préparation extemporanée d'une suspension.

3. Une composition pharmaceutique solide selon n'importe quelle des revendications ci-avant, **caractérisée en ce qu'**elle comporte aussi un ou plusieurs des excipients suivants: Kollidon CL, Kollidon VA 64, fécule de maïs, laurylsulfate de sodium, Tablettose 80, fécule 1500, stéarate de magnésium, monohydrate de lactose, PVP K30.

4. Une composition pharmaceutique solide selon n'importe quelle des revendications ci-avant, **caractérisée en ce qu'**elle comprend aussi un ou plusieurs des excipients suivants: conservateurs, édulcorants, aromatisants et colorants.

5. Un procédé pour la fabrication d'une composition pharmaceutique solide selon la revendication 1, **caractérisé en ce qu'**il comporte une phase de malaxage, dans laquelle le temps d'addition du solvant de granulation est plus grand que 4 minutes, dans laquelle la vitesse d'addition du solvant de granulation se trouve dans une plage d'entre 300 mg/minute environ et 500 mg/minute environ et où le rapport solvant de granulation/composant sec du granulé se trouve dans une plage d'entre 20 % environ et 60 % environ p/p.

6. Un procédé pour la fabrication d'une composition pharmaceutique solide selon la revendication 5, **caractérisé en ce que** dans la phase de malaxage, le rapport solvant de granulation/composant sec du granulé se trouve dans une plage d'entre 35 % environ et 40 % environ p/p.

7. Un procédé pour la fabrication d'une composition pharmaceutique solide selon la revendication 5, **caractérisé en ce que** la phase de malaxage est réalisée dans une période de 5 minutes environ à 7 minutes environ.

8. Un procédé pour la fabrication d'une composition pharmaceutique solide selon la revendication 5, **caractérisé en ce qu'**un mélange d'éthanol/eau 70-30 p/p, dans lequel l'agglutinant a été dissous au préalable, est utilisé comme solvant de granulation.

9. Un procédé pour la fabrication d'une composition pharmaceutique solide selon la revendication 8, **caractérisé en ce qu'**un mélange d'éthanol/eau 70/30 p/p, dans lequel du Kollidon 64 a été dissous au préalable, est utilisé comme solvant de granulation.

10. Une composition pharmaceutique solide selon n'importe quelle des revendications 1 à 4, **caractérisée en ce qu'**elle comporte du ritonavir en combinaison avec au moins une substance active ayant un effet thérapeutique, laquelle est différente du ritonavir.

11. Une composition pharmaceutique solide suivant la revendication 10, **caractérisée en ce que** ladite substance active ayant un effet thérapeutique, laquelle est différente du ritonavir, est choisie parmi les substances suivantes: lopinavir, saquinavir, indinavir, tenofovir, aprenavir, fosamprenavir, atazanavir et tripanavir.
